# EUROPEAN PATENT APPLICATION

(11) **EP 3 369 424 A1**
(43) Date of publication of application: **05.09.2018**
(21) Application number: 16859761.5
(22) Date of filing: 25.10.2016
(51) Int. Cl.: A61K 33/44, A61K 9/16, A61K 9/56, A61K 45/00, A61K 47/32, A61P 13/12

(54) **POROUS MATERIAL FORMULATION FOR DELIVERY TO LARGE INTESTINE OR LOWER SMALL INTESTINE**

(30) Priority: 26.10.2015 JP 2015209600
(71) Applicant: Daiichi Sankyo Company, Limited, Tokyo 103-8426 (JP)
(72) Inventor: MATSUMOTO, Koji, Tokyo 103-8426 (JP); YOSHITOMI, Tomomi, Tokyo 103-8426 (JP); TANAKA, Naomi, Tokyo 103-8426 (JP); YOSHIDA, Kazuhiro, Tokyo 103-8426 (JP); KASUYA, Yuji, Tokyo 103-8426 (JP); OHYA, Masaoki, Tokyo 103-8426 (JP)
(74) Representative: Wallace, Sheila Jane
(86) International application number: PCT/JP2016/081509
(87) International publication number: WO 2017/073531

(57) **Abstract**

The problem with which the present invention is concerned is providing a novel pharmaceutical preparation, the restrictions on the administration of which to a patient are more relaxed than those of a conventional spherical carbon adsorbent, which can be simultaneously taken with other drugs and, moreover, which is reduced in dosage. The problem can be solved by a pharmaceutical preparation for oral administration comprising a porous material, wherein a means for exposing a surface of the porous material for the first time in the large intestine or a lower part of the small intestine is provided on the porous material.

## Description

### Technical Field

The present invention relates to a pharmaceutical preparation for delivery of a porous material effective for reducing blood uremic toxins, improving uremic symptoms, delaying dialysis initiation, or protecting renal functions in chronic kidney disease (CKD) patients to the large intestine or a lower part of the small intestine.

### Background Art

Chronic kidney disease is a serious pathological condition relating to end stage renal disease (dialysis transition and renal transplantation), cardiovascular disease, and even death. The number of dialysis patients in Japan exceeds 310000, and is still increasing. Although the number of clinics with a dialysis device is becoming sufficient especially in Japan, dialysis itself, including three visits to a clinic per week, is a time-consuming burden. Also, dialysis imposes a large burden in terms of medical economy as well. Moreover, the number of donors for renal transplantation is limited. Accordingly, it is becoming very important to delay dialysis transition in predialysis CKD patients and, moreover, provide time until a renal transplantation donor appears. For current drug therapy for patients with chronic kidney disease, renin-angiotensin-based inhibitors including angiotensin II receptor blockers (ARBs) and angiotensin converting enzyme (ACE) inhibitors are first-line drugs, calcium antagonists and diuretics are second or third-line drugs, and, moreover, based on the complications and primary diseases, a large number of oral drugs are prescribed such as hyperuricemia drugs, hyperlipidemia drugs, diabetes drugs, steroids/immunosuppressants, antiplatelet drugs/anticoagulants, hyperphosphatemia drugs, erythropoietic stimulating agents, analgesics, antiarrhythmic drugs, antidepressants, Alzheimer-type dementia drugs, Parkinson's disease drugs, proton pump inhibitors (PPIs), antiallergic drugs, and antibacterial drugs. It is known that as the renal functions deteriorate, the uremic concentration in the body increases and, accordingly, uremic symptoms such as fatigue, anorexia, insomnia, pruritus, and nausea are developed (Non-Patent Literature 1). Alleviating such symptoms leads to an improved QOL of a patient.

Spherical carbon adsorbent is prescribed to predialysis CKD patients who are diagnosed with progressive CKD in order to improve uremic symptoms and delay dialysis initiation. It is known that uremic symptoms such as anorexia, bad breath, and nausea caused by the accumulation of uremic toxins in the body due to impaired renal functions are improved by taking spherical carbon adsorbent (Non-Patent Literature 2). In addition, it is known that some uremic toxins, such as indoxyl sulfate originating from indole produced by intestinal bacteria in the caecum/large intestine, actively deteriorate renal functions, and adsorption of such uremic toxins in the lumen of the gastro-intestinal (GI) tract reduces the amount of uremic toxins absorbed into the body, which makes it possible to suppress progression of renal dysfunction (decrease in eGFR and increase in serum creatinine level and BUN) of predialysis CKD patients and to delay transition to dialysis(Non-Patent Literature 3). It is expected that reduction of uremic toxins improves vascular endothelial functions and suppresses calcification (Non-Patent Literature 4), and moreover it is expected that increasing the response of C.E.R.A. (continuous EPO receptor activator) can contribute to treating or preventing anemia (Non-Patent Literature 5).

In Japan, capsule preparations and fine granule preparations of spherical carbon adsorbent and kinds of spherical carbon adsorbent preparations having brand names "Kremezin(R) Capsule 200 mg" and "Kremezin(R) Fine Granules 2 g" are commercially available. Attempts have been made to form tablet preparations in order to reduce the volume of Kremezin (Patent Literature 1), but practical use is not achieved yet.

### Citation List

### Patent Literature

Patent Literature 1: International Publication No. WO2012/121202

### Non-Patent Literature

Non-Patent Literature 1: Vassilis Liakopoulos et al., International Urology and Nephrology 2004; 36: 437-443
Non-Patent Literature 2: Keizo Koide et al., Rinsho Hyoka (Clinical Evaluation) 1987; 15(3): 527-564
Non-Patent Literature 3: Tadao Akizawa et al., Kidney and dialysis 1998; 45(3): 373-388
Non-Patent Literature 4: Ayinuer Adijiang et al., Nephrol Dial Transplant 2008; 23: 1892-1901
Non-Patent Literature 5: I-Wen Wu et al., Nephrol Dial Transplant 2014; 29(9): 1719-1727

### Summary of Invention

### Technical Problem

Spherical carbon adsorbent is prescribed in order to improve uremic symptoms and delay dialysis initiation, but it has great constraints on a patient's medication adherence. First, depending on the types of other drugs (concomitant drugs) taken by the patient, spherical carbon adsorbent may adsorb other drugs at a stage before other drugs are absorbed into the body, thus there are limitations on the dosing timing of spherical carbon adsorbent, resulting in reduced patient compliance. A reminder is provided to avoid taking spherical carbon adsorbent simultaneously with other drugs and take spherical carbon adsorbent 30 minutes to 1 hour or more after taking other drugs. (A package insert for spherical carbon adsorbent marketed in Japan explicitly states as a precaution: "When used in combination with other drugs, avoid taking them simultaneously with this drug in view of this drug being an adsorbent.") Second, the dosage of existing spherical carbon adsorbent is large (In Japan, 2 g at a time, 3 times a day, thus 6 g per day), and it causes patients to have difficulty taking it. The regimen (usage and dosage) of Kremezin(R) (hereinafter sometimes referred to as AST-120), which is a kind of spherical carbon adsorbent preparation, is also orally administered usually in 3 divided doses for 6 g a day. In the case of a capsule preparation, 10 capsules have to be taken at a time. In addition, 2 g of a fine granule preparation is not a small volume. It is known that a capsule preparation has a dead volume other than the volume of spherical carbon adsorbent. Thus, in addition to the dosage being large, the volume of the capsule itself has to be increased. Moreover, for either a capsule or a fine granule preparation, a large amount of water is required to take a single dose, thus it causes a burden on CKD patients who have a restriction on the amount of water intake.

Accordingly, an object of the present invention is to provide a novel pharmaceutical preparation having an alleviated dosing regimen, acceptability for taking other drugs simultaneously, reduced dosing amount and increased efficacy compared to those of conventional spherical carbon adsorbent.

The present inventors focused on the adsorption performance of conventional spherical carbon adsorbent after oral administration and investigated whether there is a change in adsorption performance resulting from traveling through the GI-tract. As a result, the inventors found that when spherical carbon adsorbent is exposed to the intestinal lumen fluid, the ability to adsorb uremic toxins or its precursors is reduced, and when spherical carbon adsorbent is orally administered, the adsorption performance of spherical carbon adsorbent is reduced at a stage before spherical carbon adsorbent reaches the target caecum/large intestine where intestinal bacteria are present and where uremic toxins or its precursors are adsorbed, i.e., at the duodenum/small intestine stage. Accordingly, as a result of diligent research on physical modification of a porous material for maintaining adsorption performance in the caecum/large intestine after oral administration, the inventors found that a specific pharmaceutical preparation and modification make it possible to prepare a pharmaceutical preparation that is capable of maintaining adsorption performance also in the caecum/large intestine and does not affect the plasma concentration of other drugs when other drugs are simultaneously taken, and accomplished the present invention.

### Solution to Problem

The present invention provides a pharmaceutical preparation for delivery to the large intestine or a lower part of the small intestine of a porous material, which enables a concomitant drug to be simultaneously taken, has a higher toxic substance adsorbability than conventional porous materials, and is effective for reducing blood uremic toxins, improving uremic symptoms, delaying dialysis initiation, or protecting renal functions in a CKD patient, by controlling the release of a porous material from a capsule preparation and/or the coated state of the toxic substance adsorbing surface of the porous material in the GI tract.

Specifically, the present invention provides those features set forth below.
(1) A pharmaceutical preparation for oral administration comprising a porous material, wherein a means for exposing a surface of the porous material for the first time in the large intestine or a lower part of the small intestine is provided on the porous material.
(2) A pharmaceutical preparation for oral administration comprising a porous material, wherein an amount of an adsorbate adsorbed in an adsorption test of the pharmaceutical preparation is less than 20% in an environment having a pH of 5 or less and 60% or more in an environment having a pH of 7 or more based on an amount of an adsorbate adsorbed by a porous material not provided with the means. (Preferably the pharmaceutical preparation of (1))
(3) A pharmaceutical preparation for oral administration comprising a porous material, wherein in an adsorption test using methylene blue as an adsorbate, when the pharmaceutical preparation (an amount corresponding to 50 mg of the porous material when the pharmaceutical preparation is a granule preparation, one tablet when the pharmaceutical preparation is a tablet preparation, and an amount corresponding to one capsule when the pharmaceutical preparation is a capsule preparation) is tested in a dissolution apparatus using 500 mL of a test solution having a methylene blue concentration of 40 mg/L, an amount of the adsorbate adsorbed in 2 hours in the test solution having a pH of 1.2 is less than 50 mg per 1 g of the porous material, and an amount of the adsorbate adsorbed in 6 hours in the test solution having a pH of 7.5 is 80 mg or more per 1 g of the porous material. (Preferably, the pharmaceutical preparation of (1) or (2))
(4) A pharmaceutical preparation for oral administration comprising a porous material, wherein in an adsorption test using methylene blue as an adsorbate, when the pharmaceutical preparation is tested in a dissolution apparatus using 500 mL of a test solution having a methylene blue concentration of 40 mg/L, an amount of the adsorbate adsorbed in 2 hours in the test solution having a pH of 1.2 is less than 30 mg per 1 g of the porous material, and an amount of the adsorbate adsorbed in 6 hours in the test solution having a pH of 7.5 is 100 mg or more per 1 g of the porous material. (Preferably, the pharmaceutical preparation of (1) or (2))
(5) A pharmaceutical preparation for oral administration comprising a porous material, which is a pharmaceutical preparation selected from a) to c) below:
   a) a granule preparation, a capsule preparation, or a tablet preparation comprising a porous material coated with an enteric polymer;
   b) a capsule preparation wherein a porous material is encapsulated within an enteric capsule; and
   c) a tablet preparation wherein a compression-molded product of a porous material is coated with an enteric polymer. (Preferably, the pharmaceutical preparation according to any of (1) to (4))
(6) A pharmaceutical preparation according to any one of (1) to (5), wherein the porous material is an oral adsorbent for a harmful substance.
(7) A preparation according to any one of (1) to (6), wherein the porous material is spherical carbon adsorbent.
(8) A pharmaceutical preparation according to any one of (1) to (7), wherein before the means is provided, the porous material has an average particle size X50 of 100 to 500 µm as measured by laser diffractometry and a specific surface area of 800 m²/g or more as measured by a BET multipoint method.
(9) A pharmaceutical preparation according to any one of (1) to (8), wherein before the means is provided, the porous material has an average particle size X50 of 200 to 400 µm as measured by laser diffractometry and a specific surface area of 1000 to 1700 m²/g as measured by a BET multipoint method.
(10) A pharmaceutical preparation according to any one of (1) to (9), wherein the enteric polymer or the enteric capsule dissolves at a pH of 5 to 8.
(11) A pharmaceutical preparation according to any one of (1) to (10), wherein the enteric polymer or the enteric capsule dissolves at a pH of 5 to 7.
(12) A pharmaceutical preparation according to any one of claims (1) to (11), wherein the enteric polymer is an acrylic-based polymer or a cellulose-based polymer.
(13) A pharmaceutical preparation according to any one of (1) to (12), wherein the enteric polymer is one or more selected from the group consisting of a copolymer of methacrylic acid and ethyl acrylate, a copolymer of methacrylic acid and methyl methacrylate, a copolymer of methacrylic acid, methyl acrylate, and methyl methacrylate, cellulose acetate phthalate, cellulose acetate trimellitate, hydroxypropyl methylcellulose phthalate, hydroxypropyl methylcellulose acetate succinate, carboxymethyl ethylcellulose, cellulose acetate succinate, methylcellulose phthalate, hydroxymethylcellulose phthalate, hydroxypropyl methyl acetate maleate, hydroxypropyl methyl trimellitate, polyvinyl acetate phthalate, and polyvinyl butyrate phthalate.
(14) A pharmaceutical preparation according to any one of (1) to (13), wherein the enteric polymer is one or more selected from the group consisting of 1) a copolymer of methacrylic acid and ethyl acrylate, 2) a copolymer of methacrylic acid and methyl methacrylate, 3) a copolymer of methacrylic acid, methyl acrylate, and methyl methacrylate, and 4) hydroxypropyl methylcellulose acetate succinate.
(15) A pharmaceutical preparation according to any one of (1) to (14), wherein the enteric polymer is one or more selected from methacrylic acid copolymer L, methacrylic acid copolymer S, methacrylic acid copolymer LD, and hydroxypropyl methylcellulose acetate succinate.
(16) A pharmaceutical preparation according to any one of (1) to (15), wherein the pharmaceutical preparation wherein the means for exposing a surface of the porous material for the first time in the large intestine or a lower part of the small intestine is provided is a) a granule preparation, a capsule preparation, or a tablet preparation comprising a porous material coated with an enteric polymer.
(17) A pharmaceutical preparation according to (16), wherein a coating of the enteric polymer is 10 to 100% by weight based on the porous material.
(18) A pharmaceutical preparation according to (16), wherein a coating of the enteric polymer is 10 to 60% by weight based on the porous material.
(19) A pharmaceutical preparation according to (16), wherein a coating of the enteric polymer is 30 to 60% by weight based on the porous material.
(20) A pharmaceutical preparation according to any one of (1) to (19), wherein
   the pharmaceutical preparation wherein the means for exposing a surface of the porous material for the first time in the large intestine or a lower part of the small intestine is provided is a granule preparation or a capsule preparation comprising a porous material coated with an enteric polymer;
   the porous material is spherical carbon adsorbent;
   the enteric polymer is one or more selected from the group consisting of methacrylic acid copolymer L, methacrylic acid copolymer S, methacrylic acid copolymer LD, and hydroxypropyl methylcellulose acetate succinate; and
   an amount of a coating of the enteric polymer is 10 to 60% by weight based on the porous material.
(21) A pharmaceutical preparation according to any one of (1) to (20), wherein
   the pharmaceutical preparation wherein the means for exposing a surface of the porous material for the first time in the large intestine or a lower part of the small intestine is provided is a granule preparation or a capsule preparation comprising a porous material coated with an enteric polymer;
   the porous material is spherical carbon adsorbent;
   the enteric polymer is one or more selected from the group consisting of methacrylic acid copolymer L and methacrylic acid copolymer S; and
   an amount of a coating of the enteric polymer is 10 to 60% by weight based on the porous material.
(22) A pharmaceutical preparation according to any one of (1) to (21), for reducing a blood uremic toxin, improving a uremic symptom, delaying dialysis initiation, or protecting a renal function in a patient with CKD.
(23) A pharmaceutical preparation according to any one of (1) to (22), for simultaneous administration with a further drug.
(24) A pharmaceutical preparation according to any one of (1) to (23), not affecting a blood level of a further drug when simultaneously administered with the further drug.
(25) A pharmaceutical preparation according to any one of (1) to (24), further comprising a further drug.
(26) A pharmaceutical preparation according to any one of (23) to (25), wherein the further drug is one or more selected from angiotensin II receptor blockers, angiotensin converting enzyme inhibitors, calcium antagonists, diuretics, hyperuricemia drugs, hyperlipidemia drugs, diabetes drugs, steroid/immunosuppressants, antiplatelet drugs/anticoagulants, hyperphosphatemia drugs, erythropoietic stimulating agents, analgesics, antiarrhythmic drugs, antidepressants, Alzheimer-type dementia drugs, Parkinson's disease drugs, proton pump inhibitors (PPIs), antiallergic drugs, and antibacterial drugs.
(27) A pharmaceutical preparation according to any one of (23) to (25), wherein the further drug is one or more selected from angiotensin II receptor blockers, angiotensin converting enzyme inhibitors, calcium antagonists, diuretics, and antiplatelet drugs/anticoagulants.
(28) A method for producing the pharmaceutical preparation for oral administration of (1) to (27), the method comprising any of the following steps:
   a) a step of coating a porous material with an enteric polymer;
   b) a step of encapsulating a porous material within an enteric capsule; and
   c) a step of coating a compression-molded product of a porous material with an enteric polymer.

Also, the present invention provides those features set forth below.
(1) A pharmaceutical preparation for oral administration, wherein a means for exposing for the first time in the large intestine or a lower part of the small intestine is provided on a porous material.
(2) A pharmaceutical preparation according to (1), wherein the means for exposing for the first time in the large intestine or a lower part of the small intestine is coating of the porous material with an enteric polymer.
(3) A pharmaceutical preparation according to (1), wherein the means for exposing for the first time in the large intestine or a lower part of the small intestine is encapsulation of the porous material that may be coated with an enteric polymer within an enteric capsule.
(4) A pharmaceutical preparation according to (2) or (3), wherein the enteric polymer is a cellulose-based, vinyl-based, or acrylic-based polymer.
(5) A pharmaceutical preparation according to (2) or (3), wherein the enteric polymer is an acrylic-based polymer.
(6) A pharmaceutical preparation according to (2) or (3), wherein the enteric polymer is an acrylic-based polymer, and a coating of the acrylic-based polymer is 10 to 60% by weight based on the porous material.
(7) A pharmaceutical preparation according to (2) or (3), wherein the enteric polymer is an acrylic-based polymer, and a coating of the acrylic-based polymer is 20 to 40% by weight based on the porous material.
(8) A pharmaceutical preparation according to any one of (5) to (7), wherein the acrylic-based polymer is one or more selected from a copolymer of methacrylic acid and ethyl acrylate, a copolymer of methacrylic acid and methyl methacrylate, and a copolymer of methacrylic acid, methyl acrylate, and methyl methacrylate.
(9) A pharmaceutical preparation according to any one of (5) to (7), wherein the acrylic-based polymer is one or more selected from methacrylic acid copolymer L and methacrylic acid copolymer S.
(10) A pharmaceutical preparation according to any one of (1) to (9), wherein the porous material is spherical carbon adsorbent.
(11) A pharmaceutical preparation according to any one of (1) to (9), wherein the porous material is bulk Kremezin (spherical carbon adsorbent obtained by oxidizing and reducing, at high temperature, spherical fine particle porous carbon derived from petroleum hydrocarbon).
(12) A pharmaceutical preparation according to any one of (1) to (11), for improving a uremic symptom or delaying dialysis initiation in a patient with CKD.
(13) A pharmaceutical preparation according to any one of (1) to (12), for simultaneous administration with a further drug.
(14) A pharmaceutical preparation according to any one of claims (1) to (13), further comprising a further drug.
(15) A pharmaceutical preparation according to (13) or (14), wherein the further drug is one or more selected from angiotensin II receptor blockers, angiotensin converting enzyme inhibitors, calcium antagonists, diuretics, hyperuricemia drugs, hyperlipidemia drugs, diabetes drugs, steroids/immunosuppressants, antiplatelet drugs/anticoagulants, hyperphosphatemia drugs, erythropoietic stimulating agents, analgesics, antiarrhythmic drugs, antidepressants, Alzheimer-type dementia drugs, Parkinson's disease drugs, proton pump inhibitors (PPIs), antiallergic drugs, and antibacterial drugs.
(16) A pharmaceutical preparation according to (13) or (14), wherein the further drug is one or more selected from angiotensin II receptor blockers, angiotensin converting enzyme inhibitors, calcium antagonists, and diuretics.
(17) A method for producing a pharmaceutical preparation for oral administration wherein a means for exposing for the first time in the large intestine or a lower part of the small intestine is provided on a porous material, the method comprising a step of coating the porous material with an enteric polymer.
(18) A method for producing a pharmaceutical preparation for oral administration wherein a means for exposing for the first time in the large intestine or a lower part of the small intestine is provided on a porous material, the method comprising a step of encapsulating the porous material that may be coated with an enteric polymer within an enteric capsule.

### Advantageous Effects of Invention

The pharmaceutical preparation for oral administration of a porous material of the present invention has excellent simultaneous usability with other drugs, contributes to excellent medication compliance, and is useful for reducing blood uremic toxins, improving uremic symptoms, delaying dialysis initiation, protecting renal functions, or the like in a patient with CKD.

### Brief Description of Drawings

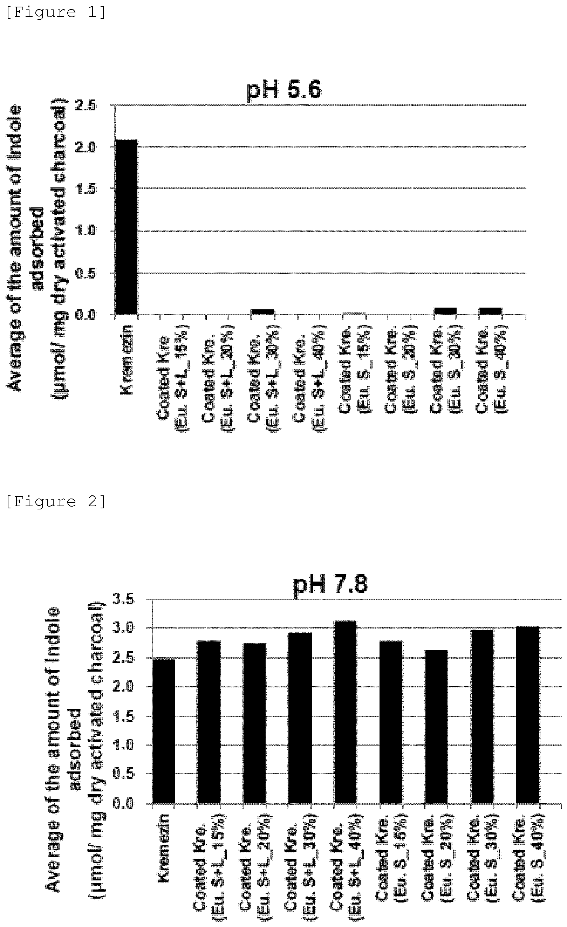

[Figure 1] Figure 1 is a graph showing the results of the adsorption performance evaluation test (pH 5.6 conditions) of Example 4 on the granule preparations (Formulation Examples 8 to 11 and 4 to 7) of the present invention. "Kremezin" indicates uncoated AST-120, "S+L" indicates the granule preparations of Example 3 (Formulation Examples 8 to 11, Eudragit S100 and Eudragit L100 (mixed in a ratio of 1:1) used as enteric polymers), "S" indicates the granule preparations of Example 3 (Formulation Examples 4 to 7, Eudragit S100 used as an enteric polymer), and % indicates the weight-based proportion of enteric polymer solids to AST-120 in the granule preparations.
[Figure 2] Figure 2 is a graph showing the results of the adsorption performance evaluation test (pH 7.8 conditions) of Example 4 on the granule preparations (Formulation Examples 8 to 11 and 4 to 7) of the present invention. "Kremezin", "S+L", "S", and "%" are the same as those in Figure 1 described above.
[Figure 3] Figure 3 is a graph showing the results of an adsorption performance evaluation test (pH 5.8 conditions) of Example 4 on the granule preparations (Formulation Examples 12 to 14) of the present invention.
[Figure 4] Figure 4 is a graph showing the results of the adsorption performance evaluation test (pH 7.7 conditions) of Example 4 on the granule preparations (Formulation Examples 12 to 14) of the present invention.

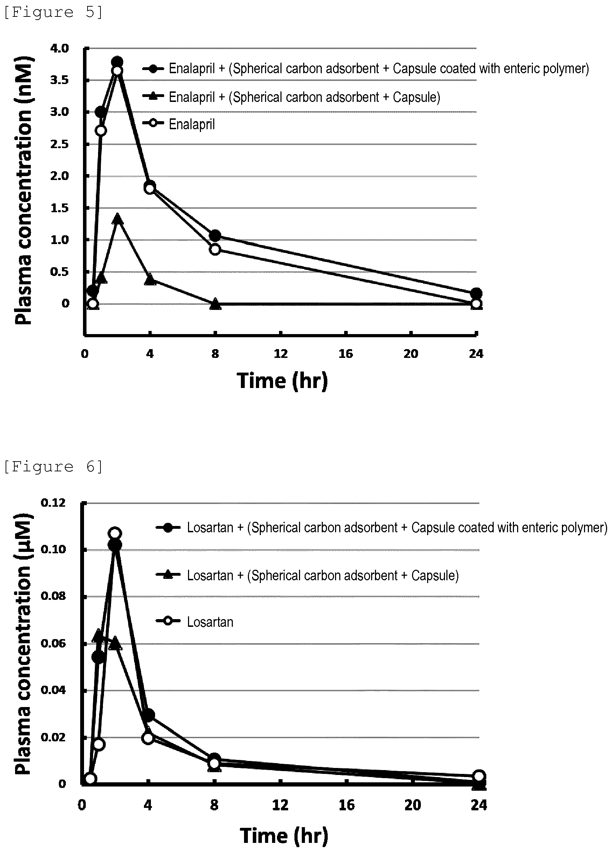

[Figure 5] Figure 5 is a graph showing the results of the in vivo evaluation test of Example 5 on the capsule preparation of the present invention (a change in blood level of enalaprilat (an active metabolite of enalapril) when the capsule preparation is simultaneously administered with enalapril). The black circle symbol indicates the value of a group administered with an enalapril-containing capsule preparation (Formulation Example 2) produced in Example 2, the black triangle symbol indicates the value of a control group (a group administered with an enalapril-containing capsule preparation as produced in Example 2 except that a size-5 capsule not coated with an enteric polymer is filled with spherical carbon adsorbent), and the white circle symbol indicates the value of a group administered solely with enalapril (one size-0 capsule filled solely with enalapril).
[Figure 6] Figure 6 is a graph showing the results of the in vivo evaluation test of Example 6 on the capsule preparation of the present invention (a change in blood level of losartan when the capsule preparation is simultaneously administered with losartan). The black circle symbol indicates the value of a group administered with a losartan-containing capsule preparation (Formulation Example 3) produced in Example 2, the black triangle symbol indicates the value of a control group (a group administered with a losartan-containing capsule preparation as produced in Example 2 except that a size-5 capsule not coated with an enteric polymer is filled with spherical carbon adsorbent), and the white circle symbol indicates the value of a group administered solely with losartan (one size-0 capsule filled solely with losartan).

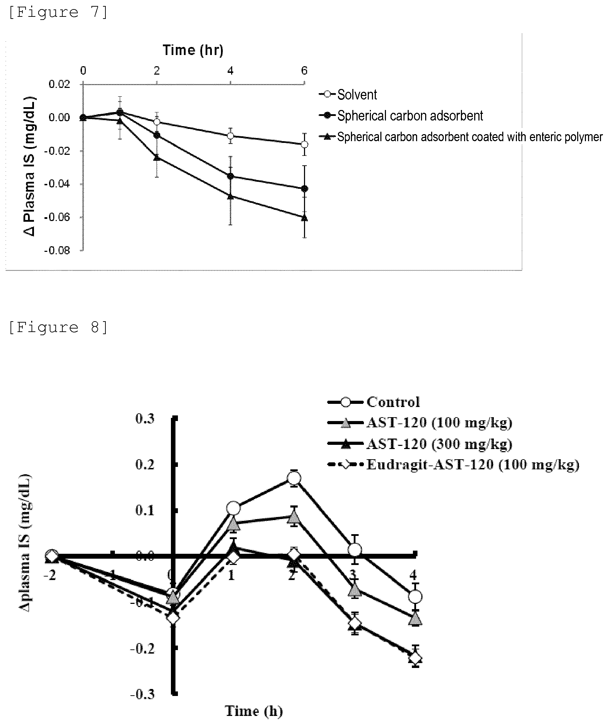

[Figure 7] Figure 7 is a graph showing the results (action to reduce uremic toxins in plasma) of the in vivo evaluation test of Example 7 on the granule preparation of the present invention (Formulation Example 7). The black triangle symbol indicates the value of a group administered with the granule preparation of Example 3 (Formulation Example 7: a pharmaceutical preparation wherein a coating of Eudragit S100 as an enteric polymer is provided to a ratio of 40% by weight relative to AST-120), the black circle symbol indicates the value of a group administered with uncoated AST-120, and the white circle symbol indicates the value of a solvent-administered group.
[Figure 8] Figure 8 is a graph showing the results (action to reduce uremic toxin level in plasma) of the in vivo evaluation test of Example 8 on the granule preparation of the present invention (Formulation Example 12) .
[Figure 9] Figure 9 is a graph showing the results (action to reduce uremic toxin level in plasma; Δ plasma IS, mg/dL) of the in vivo evaluation test of Example 9 wherein a capsule was filled with the granule preparation of the present invention (Formulation Example 12) and administered to a dog.
[Figure 10] Figure 10 is a graph showing the results (action to reduce uremic toxin level in plasma; Δ plasma IS AUC (0 to 8 h), mg·h/dL) of the in vivo evaluation test of Example 9 wherein a capsule was filled with the granule preparation of the present invention (Formulation Example 12) and administered to a dog.
[Figure 11] Figure 11 is a photo showing the state of the granule preparation of the present invention (Formulation Example 7) in the small intestine 1 hour after administration in the in vivo evaluation test of Example 11 on the granule preparation.
[Figure 12] Figure 12 is a photo showing the state of the granule preparation of the present invention (Formulation Example 7) in the large intestine 4 hours after administration in the in vivo evaluation test of Example 11 on the granule preparation.
[Figure 13] Figure 13 is a graph showing the results (influence on blood warfarin level) of the in vivo evaluation test of Example 12 when a capsule filled with the granule preparation of the present invention (Formulation Example 12) together with warfarin was administered.

### Description of Embodiment

In the present invention, the "porous material" refers to a functional substance having small pores inside the structure of a solid. The porous material before being provided with a means for exposing the surface for the first time in the large intestine or a lower part of the small intestine has a specific surface area (a BET multipoint method) of 800 or more (preferably 1000 to 1700) (m²/g) and an average particle size (laser diffractometry) X50 of 100 to 500 (preferably 200 to 400) (µm). More preferably, the porous material has a specific surface area (a BET multipoint method) of 1200 to 1600 (m²/g) and an average particle size (laser diffractometry) X50 of 300 to 380 (µm). The specific surface area (a BET multipoint method) and the average particle size (laser diffractometry) can be measured in accordance with the Japanese Pharmacopoeia 17th edition.

Examples of porous materials usable in the present invention include activated carbon (including spherical carbon adsorbent) and zeolite. In general, porous materials are functional substances. For example, zeolite, which is called boiling stone, is applied to deodorization, moisture absorption, water absorption, and water purification, and is used as a catalyst and a separation material in the petrochemical field. Activated carbon is used in purification and deodorization of water in the environmental field. In addition to zeolite and activated carbon, it is expected in recent years that PCP (porous coordination polymer)/MOF (metal-organic framework) as novel porous materials are applied to, for example, selective storage and sustained release of molecules and ions. In the present invention, the function of a porous material means, in particular, being capable of demonstrating the ability to adsorb the target adsorbates (such as uremic toxins, phosphorus in the case of hyperphosphatemia, and potassium in the case of hyperkalemia) under physiological conditions as an oral adsorbent that can ensure efficacy as a pharmaceutical product. The present invention is applicable to a porous material having the properties of being capable of significantly adsorbing uremic toxins existing in a concentration of several grams/liter when added in an amount of several grams/liter or less that is possible by administration as a pharmaceutical product in a medium having the properties of the fluid of the digestive tract, as indicated in a known publication (Miyazaki et al., Japanese Journal of Pharmaceutical Health Care and Sciences 2008; 34, 1077-1085) or the like.

As the porous material used in the present invention, those that are commercially available or known, or produced by a known production method can be used. For example, activated carbon as one example of the porous material can be produced by carbonizing and activating a spherical phenol resin through heat treatment under predetermined conditions, washing and reheating the resin by predetermined methods, and sieving the resin as necessary, as indicated in Japanese Patent No. 3585043. The physicochemical properties of the porous material used in the present invention can be specified primarily by the specific surface area, the pore volume, and the packing density. For example, Japanese Patent No. 3585043 shows characteristics values such as specific surface area: 800 to 2000 m²/g, pore volume: 0.2 to 1.0 mL/g, packing density: 0.5 to 0.75 g/mL, pore diameter: 1.7 to 2.0 nm, maximum particle size: 425 µm or less, and average particle size: 350 µm or less. Japanese Patent No. 5124094 shows characteristics values such as specific surface area: 1150 to 1500 m²/g, pore volume: 0.2 to 1.0 mL/g, packing density: 0.5 to 0.7 g/mL, maximum particle size: 425 µm or less, and average particle size: 350 µm or less (Japanese Patent No. 5124094). Examples of other porous materials for use in the present invention include medicinal carbon set forth in the Japanese Pharmacopoeia, rice husk carbon or rice straw carbon containing amorphous silica (Japanese Patent Laid-Open No. 2014-181144), organic porous bodies (Japanese Patent Laid-Open No. 2014-77138), and PCP/MOF (Hirayasu Furukawa et al., Science 2010; 329: 424-428, Alexandra M.Z. Slawin et al., Angew. Chem. Int. Ed. 2010; 49: 8630-8634). Network/porous polymers, resins, and the like used in therapeutic agents for hyperphosphatemia and hyperkalemia can be used as well.

In the present invention, "spherical carbon adsorbent" is a spherical microparticulate porous material primarily composed of carbon. In Japan, capsule preparations and fine granule preparations of spherical carbon adsorbent are commercially available, and AST-120 (product name in Japan "Kremezin(R)") is also carbon adsorbent of spherical fine particles composed of high-purity porous carbon for which petroleum pitch is used as an ingredient.

In the present invention, the phrase "for exposing a surface of the porous material for the first time in the large intestine or a lower part of the small intestine" means that the surface is covered with an enteric polymer or within an enteric capsule before oral administration, and after administration, the surface is substantially covered before the large intestine or a lower part of the small intestine, which is the target site, is reached, and uncovered when the target site is reached. It is known that the pH in the stomach fluctuates between 1 and 5 during the day, and the pH fluctuates between 5 and 6 in an upper part of the small intestine including the duodenum. Accordingly, the phrase "the surface is substantially covered before the large intestine or a lower part of the small intestine, which is the target site, is reached" means that the adsorption performance for uremic toxins and precursors thereof or concomitantly used other drugs in an acidic to weakly acidic environment is suppressed to less than 40% and preferably less than 20% compared with a porous material that is not provided with the means. The phrase "uncovered when the target site is reached" means that the adsorption performance of the porous material is recovered (reactivated) in a neutral environment. When the porous material (carbon adsorbent) is coated with an enteric polymer, carbon adsorbent (black) covered with a white enteric polymer appears white. After this carbon adsorbent is orally administered to rats, the rats are euthanized over time to observe with the naked eye whether the carbon adsorbent present in the lumen of the digestive tract has returned to black, and it is thereby possible to verify whether the surface is exposed (see Example 11 described below). Also, by performing an adsorption test on uremic toxins and precursors thereof or expected concomitantly used other drugs in an acidic to weakly acidic environment and a neutral environment, measuring the amounts of these substances adsorbed, and making a comparison with the amounts of these substances adsorbed by a porous material that is not covered with an enteric polymer or within an enteric capsule, it is possible to verify whether the amounts of these substances adsorbed are suppressed or recovered (reactivated) (see Example 4 and Example 13 described below).

Preferably, the pharmaceutical preparation of the present invention is "a pharmaceutical preparation, wherein an amount of an adsorbate adsorbed in an adsorption test of the pharmaceutical preparation is less than 40% and preferably less than 20% in an environment having a pH of 5 or less, and 50% or more and preferably 60% or more in an environment having a pH of 7 or more based on an amount of an adsorbate adsorbed by a porous material not provided with the means". Here, indoxyl sulfate, indole, methylene blue, and the like are used as adsorbates. The adsorption test can be performed by the following adsorption test method using methylene blue or the method of Example 4 or 13 described below.

Preferably, the pharmaceutical preparation of the present invention is "a pharmaceutical preparation, wherein in an adsorption test using methylene blue as an adsorbate, when the pharmaceutical preparation (an amount corresponding to 50 mg of the porous material when the pharmaceutical preparation is a granule preparation, one tablet when the pharmaceutical preparation is a tablet preparation, and an amount corresponding to one capsule when the pharmaceutical preparation is a capsule preparation) is tested in a dissolution apparatus using 500 mL of a test solution having a methylene blue concentration of 40 mg/L, an amount of the adsorbate adsorbed in 2 hours in the test solution having a pH of 1.2 is less than 50 mg (preferably less than 30 mg) per 1 g of the porous material, and an amount of the adsorbate adsorbed in 6 hours in the test solution having a pH of 7.5 is 80 mg or more (preferably 100 mg or more) per 1 g of the porous material". This adsorption test is performed by the following method.

### (Adsorption test method)

Evaluations are made using a sample (an amount corresponding to 50 mg in terms of the weight of the porous material when the pharmaceutical preparation is a granule preparation containing the porous material coated with an enteric polymer, one tablet when the pharmaceutical preparation is a tablet preparation, one capsule when the capsule itself is enteric, and an amount corresponding to one capsule obtained by taking out the contents when the capsule is an ordinary non-enteric capsule and the contents are coated with an enteric polymer).

A test solution having a pH of 1.2 (such as Japanese Pharmacopoeia dissolution test 1st solution JP1) or a pH of 7.5 (such as McIlvaine buffer) is used. The test solution is adjusted such that the concentration of methylene blue, which is an adsorbate, is 40 mg/L. The methylene blue absorbance is measured using a dissolution apparatus by a paddle method at 200 rpm in 500 mL of a test solution at 246 nm of UV measurement wavelength (the test time is 2 hours at a pH of 1.2 and 6 hours at a pH of 7.5). In reference to the JIS activated carbon test, the amount of methylene blue adsorbed on 1 g of spherical carbon adsorbent is calculated. (An example of a test performed on the pharmaceutical preparation of the present invention is shown in Example 13).

The pharmaceutical preparation of the present invention, because the porous material is isolated from the environment of the digestive tract until the target site (the large intestine or a lower part of the small intestine) is reached, functions to maintain the intrinsic adsorption performance without being reduced and demonstrate the adsorption performance of the porous material in the target site.

Examples of the "means for exposing a surface of the porous material for the first time in the large intestine or a lower part of the small intestine" or the "means for exposing for the first time in the large intestine or a lower part of the small intestine" used in the present invention include enteric release preparations, timed release preparations, sustained release preparations, or large-intestine release preparations using an intestinal bacteria soluble polymer such as chitosan, in which an enteric base that dissolves site-specifically in the digestive tract from a lower part of the small intestine to the large intestine is used, and dosage forms such as tablet preparations, capsule preparations, and granule preparations are considered. Among these, in particular, enteric release preparations, timed release preparations, and large-intestine release preparations using an intestinal bacteria soluble polymer such as chitosan are techniques enabling drug delivery specifically to the large intestine or a lower part of the small intestine according to the properties and the amount of the polymer used.

Particularly preferred embodiments of the "means for exposing for the first time in the large intestine or a lower part of the small intestine" used in the present invention include 1) coating a porous material with an enteric polymer, and 2) encapsulating a porous material that may be coated with an enteric polymer within an enteric capsule.

Embodiments of the "pharmaceutical preparation, wherein the means for exposing a surface of the porous material for the first time in the large intestine or a lower part of the small intestine is provided" of the present invention include:
a) a granule preparation, a capsule preparation, or a tablet preparation comprising a porous material coated with an enteric polymer;
b) a capsule preparation wherein a porous material is encapsulated within an enteric capsule; and
c) a tablet preparation wherein a compression-molded product of a porous material is coated with an enteric polymer.

The "granule preparation" of the present invention includes granule preparations, fine granule preparations, or powder preparations set forth in the Japanese Pharmacopoeia.

It is preferred that the enteric polymer or the enteric capsule used in the present invention dissolves at a pH of 5 to 8, and preferably at a pH of 5 to 7.

The "enteric polymer" used in the present invention is not particularly limited, and examples include acrylic-based, cellulose-based, and vinyl-based polymers.

Examples of the acrylic-based polymer include 1) a copolymer of methacrylic acid and ethyl acrylate, 2) a copolymer of methacrylic acid and methyl methacrylate, or 3) a copolymer of methacrylic acid, methyl acrylate, and methyl methacrylate. 1) The copolymer of methacrylic acid and ethyl acrylate is listed in the Japanese Pharmacopoeia as methacrylic acid copolymer LD, and is commercially available from Evonik Degussa Japan under product name Eudragit L30D-55. 2) The copolymer of methacrylic acid and methyl methacrylate is described in the "Japanese Pharmaceutical Excipients" as a representative enteric polymer. Those having a methacrylic acid content of 27.6% to 30.7% (hereinafter also referred to as methacrylic acid copolymer S) and 46.0 to 50.6% (hereinafter also referred to as methacrylic acid copolymer L) are particularly preferred as the enteric polymer of the present invention. Methacrylic acid copolymer L and methacrylic acid copolymer S are commercially available from Evonik Degussa Japan under product names: Eudragit L100 (methacrylic acid copolymer L) and Eudragit S100 (methacrylic acid copolymer S). 3) The copolymer of methacrylic acid, methyl acrylate, and methyl methacrylate is commercially available from Evonik Degussa Japan under product name: Eudragit FS30D.

Examples of the cellulose-based polymer include cellulose acetate phthalate, cellulose acetate trimellitate, hydroxypropyl methylcellulose phthalate (hereinafter also referred to as hypromellose phthalate), hydroxypropyl methylcellulose acetate succinate (hereinafter also referred to as hypromellose acetate succinate or hypromellose acetic acid ester succinic acid ester), carboxymethyl ethylcellulose, cellulose acetate succinate, methylcellulose phthalate, hydroxymethyl cellulose phthalate, hydroxypropyl methyl acetate maleate, and hydroxypropyl methyl trimellitate. Among these, cellulose acetate phthalate, hypromellose phthalate, and hypromellose acetic acid ester succinic acid ester are preferred. Hypromellose acetic acid ester succinic acid ester is commercially available from Shin-Etsu Chemical Co., Ltd., under product name Shin-Etsu AQOAT(R) in several grades having different pH solubilities such as HPMC-AS-HG.

Examples of the vinyl-based polymer include polyvinyl acetate phthalate and polyvinyl butyrate phthalate.

Specific examples of particularly preferred enteric polymers include methacrylic acid copolymer L, methacrylic acid copolymer S, methacrylic acid copolymer LD, and hydroxypropyl methylcellulose acetate succinate.

Two or more enteric polymers can be used in combination in an arbitrary proportion. In particular, it is preferred to combine enteric polymers such that the polymers dissolve at a pH of 5 to 8, and preferably a pH of 5 to 7. A combination of methacrylic acid copolymer L and methacrylic acid copolymer S is particularly preferred, and it is preferred to use methacrylic acid copolymer S in an amount of 0.3 to 5 times and preferably in an amount of 1 to 3 times methacrylic acid copolymer L in terms of weight ratio.

The enteric polymer is combined with a lubricant, a plasticizer, a pigment, and the like as necessary, and is used for coating. Examples of the lubricant to be added include talc, and examples of the plasticizer include triethyl citrate.

As a procedure for coating a porous material with an enteric polymer, a widely used procedure for coating fine particles can be used. As apparatuses for use in coating fine particles, for example, a composite fluidized bed granulator coater, a Wurster fluidized bed granulator coater, a tumbling fluidized bed granulator coater, and a fluidized bed granulator coater can be used.

Tablet preparations and the like can be coated with the enteric polymer by a widely used apparatus for film coating or the like.

In the present invention, the "enteric capsule" refers to a capsule that dissolves in a lower part of the small intestine or the large intestine. The enteric capsule can be produced by coating an ordinary gelatin capsule or HPMC capsule with an enteric polymer by a widely used coating procedure using a rotary pan coater, a fluidized bed granulator coater, or the like. Commercially available enteric capsules may be used as well.

Below, embodiments of the present invention will now be described together with production methods.

The first embodiment of the present pharmaceutical preparation is a granule preparation containing a porous material coated with an enteric polymer.

The second embodiment is a capsule preparation wherein the granule preparation of the first embodiment is encapsulated within an ordinary capsule.

The third embodiment is a capsule preparation wherein an uncoated porous material is encapsulated within an enteric capsule.

The fourth embodiment is a capsule preparation wherein the granule preparation of the first embodiment is encapsulated within an enteric capsule.

The fifth embodiment is a tablet preparation wherein the granule preparation of the first embodiment is compression-molded.

The sixth embodiment is a tablet preparation wherein a compression-molded product of a porous material is coated with an enteric polymer.

The seventh embodiment is a capsule preparation as in the second to fourth embodiments containing a concomitant drug.

The pharmaceutical preparation of the present invention can be produced by the following methods.

### (Production Method A)

The "granule preparation containing a porous material coated with an enteric polymer" of the first embodiment of the present invention can be produced, for example, by the following method.

A fluidized bed coater (such as Powrex FD-MP-01) can be used for coating. The porous material to be used is as described above, and, for example, spherical carbon adsorbent (such as AST-120) can be used. The enteric polymer to be used and a preferred example thereof are as described above. To the enteric polymer, it is preferred to add triethyl citrate as a plasticizer and talc as a lubricant. The enteric polymer, the plasticizer, and the lubricant are mixed with ethanol or the like to prepare a coating solution. A granule preparation can be obtained by coating a porous material with the prepared coating solution using the above fluidized bed coater.

The amount of the enteric polymer coating may be suitably arranged according to the properties of the enteric polymer so as to serve the function to expose the surface of the porous material for the first time in the large intestine or a lower part of the small intestine. Whether the function to expose the surface of the porous material for the first time in the large intestine or a lower part of the small intestine is served can be verified by the adsorption test described above or a test as performed in Example 4, 11, or 13 described below. As for a preferred range of the amount of coating, the content of the enteric polymer is preferably 10 to 100% by weight in terms of polymer solids, more preferably 10 to 60% by weight, and most preferably 30 to 60% by weight based on the porous material (such as AST-120).

### (Production Method B)

The capsule preparation of the second embodiment can be produced by encapsulating the granule preparation produced using Production Method A within a gelatin capsule or an ordinary capsule that uses a cellulose derivative, starch, or the like according to a standard method together with an additive or the like as necessary. A second drug can also be encapsulated within the capsule to form a combination drug.

### (Production Method C)

The "capsule preparation wherein an uncoated porous material is encapsulated within an enteric capsule" of the third embodiment can be produced, for example, by the following method.

A non-enteric gelatin capsule or ordinary capsule that uses a cellulose derivative, starch, or the like is coated with the enteric polymer described above using a fluidized bed coater (such as Powrex FD-MP-01) or a rotary pan coater (such as Freund HC-Lab). The amount of the coating may be suitably arranged according to the properties of the enteric polymer so as to serve the function to expose the surface of the porous material for the first time in the large intestine or a lower part of the small intestine. A subcoating can also be provided in order to improve the coating suitability of the polymer and reduce the clearance present between the cap and the body of the capsule before coating the capsule with the enteric polymer.

Commercially available enteric capsules can also be used as long as they serve the function to expose the surface of the porous material for the first time in the large intestine or a lower part of the small intestine.

Whether the enteric capsule serves the function to expose the surface of the porous material for the first time in the large intestine or a lower part of the small intestine can be verified by, for example, the adsorption test described above or a test as performed in Example 4, 11, or 13 described below).

A small capsule preparation produced by this method can be further encapsulated within a gelatin capsule or an ordinary capsule that uses a cellulose derivative, starch, or the like together with a further drug to form a combination capsule preparation as well.

### (Production Method D)

When producing the "capsule preparation wherein the granule preparation of the first embodiment is encapsulated within an enteric capsule" of the fourth embodiment, the capsule preparation may be produced by combining Production Method A and Production Method C. The amount of the coating on the porous material is adjusted according to the properties of the capsule. A further drug can be further encapsulated to form a combination capsule preparation as well.

### (Production Method E)

The tablet preparation wherein the granule preparation of the first embodiment is compression-molded of the fifth embodiment can be obtained by suitably combining the granules obtained by Production Method A with a commonly used excipient, binder, disintegrant, lubricant, and the like, and compression-molding the mixture.

### (Production Method F)

The tablet preparation wherein a compression-molded product of a porous material is coated with an enteric polymer of the sixth embodiment can be obtained by suitably combining the porous material with a commonly used excipient, binder, disintegrant, lubricant, and the like, compression-molding the mixture, and then coating the mixture with an enteric polymer using a widely used tablet coater. A subcoating can also be provided in order to improve the coating suitability of the polymer before coating with the enteric polymer.

The pharmaceutical preparation of the present invention acts to adsorb uremic toxins in a smaller amount than a conventional spherical carbon adsorbent, and therefore is useful for reducing blood uremic toxins, improving uremic symptoms, delaying dialysis initiation, or protecting renal functions in a patient with a renal disease, in particular, CKD. Examples of uremic symptoms of a patient with CKD include fatigue, anorexia, insomnia, pruritus, and nausea. It is known that some uremic toxins, such as indoxyl sulfate originating from indole produced by intestinal bacteria in the caecum/large intestine, actively deteriorate renal functions. By adsorbing such uremic toxins in the lumen of the GI tract to reduce the amount of uremic toxins absorbed into the body, the pharmaceutical preparation of the present invention can protect renal functions and suppress progression of renal dysfunction (decrease in eGFR and increase in serum creatinine level and BUN) of a patient with CKD and delay transition to dialysis. It is expected that reduction of blood uremic toxins improves vascular endothelial functions and suppresses calcification, and moreover it is expected that increasing the response of C.E.R.A. (Continuous EPO Receptor Activator) can contribute to treating or preventing anemia.

The dosage of the pharmaceutical preparation of the present invention varies according to the symptom, age, body weight, and the like. It is usually administered one to several times a day and 300 to 2000 mg and preferably 300 to 1000 mg at a time per adult in terms of the amount of the porous material, and 900 to 6000 mg and preferably 900 to 3000 mg as a daily dose.

When used in combination with a further drug, the pharmaceutical preparation of the present invention can be administered simultaneously. Examples of usable concomitant drugs include a large number of oral drugs such as cardiovascular drugs such as angiotensin II receptor blockers, angiotensin converting enzyme inhibitors, calcium antagonists, and diuretics used in drug therapy for patients with chronic kidney disease; hyperuricemia drugs, hyperlipidemia drugs, diabetes drugs, steroids/immunosuppressants, antiplatelet drugs/anticoagulants, hyperphosphatemia drugs, erythropoietic stimulating agents, analgesics, antiarrhythmic drugs, antidepressants, Alzheimer-type dementia drugs, Parkinson's disease drugs, proton pump inhibitors (PPIs), antiallergic drugs, and antibacterial drugs prescribed based on the complications and primary diseases; and OTC drugs.

"Angiotensin II receptor blockers" refer to losartan, candesartan, valsartan, telmisartan, olmesartan, irbesartan, azilsartan, and the like.

"Angiotensin converting enzyme inhibitors" refer to captopril, enalapril, alacepril, derapril, cilazapril, lisinopril, benazepril, imidapril, temocapryl, quinapril, trandolapril, perindopril erbumine, and the like.

"Calcium antagonists" refer to nifedipine, amlodipine, efonidipine, cilnidipine, nicardipine, nisoldipine, nitrendipine, nilvadipine, barnidipine, felodipine, benidipine, manidipine, azelnidipine, alanidipine, diltiazem, and the like.

"Diuretics" refer to trichlormethiazide, benzylhydrochlorothiazide, hydrochlorothiazide, methiclane, indavamide, tripamide, mefruside, furosemide, triamterene, and the like.

"Antiplatelet drugs/anticoagulants" refer to aspirin, clopidogrel, prasugrel, ticlopidine, cilostazol, ethyl icosapentate, dipyridamole, sarpogrelate, beraprost, limaprost alfadex, warfarin, dabigatran, rivaroxaban, apixaban, edoxaban, rivaroxaban, apixaban, and the like.

Also, the pharmaceutical preparation of the present invention can be formed into a combination drug with the above-described therapeutic agents to be concomitantly used. For example, a combination drug can be produced by encapsulating a porous material coated with an enteric polymer and a concomitant drug within one capsule. A combination drug can also be produced by encapsulating a porous material within an enteric capsule and further encapsulating the enteric capsule and a concomitant drug within one capsule. The proportion of a concomitant drug to be combined can be arbitrarily set, and usually the ratio of a porous material to a therapeutic agent to be concomitantly used is usually in the range of 1:0.0001 to 20 and particularly preferably in the range of 1:0.0005 to 10 in terms of weight ratio.

Next, the present invention will now be described in more detail by way of Reference Examples and Examples, but the present invention is not limited thereto.

In the Reference Examples and Examples below, the following porous materials, which are types of spherical carbon adsorbent, were used.
1) Kremezin fine granules (hereinafter sometimes referred to as AST-120 fine granules, AST-120, Kremezin, or Kre)
2) Spherical carbon adsorbent fine granules "Mylan" (hereinafter sometimes referred to as spherical carbon adsorbent "Mylan" or Merchmezin)
3) Spherical carbon adsorbent fine granules "Nichiiko" (hereinafter sometimes referred to as spherical carbon adsorbent "Nichiiko" or Kyucal)

### EXAMPLES

### (Reference Example 1) Influence of intestinal lavage fluid (intestinal lumen fluid) on adsorption performance of spherical carbon adsorbent

Male Crlj:ZUC-Lepr[fa] rats at 9 weeks old were euthanized with carbon dioxide, and then the digestive tract over a length of 60 cm from the stomach pylorus (duodenum) (about 30 cm remaining to the caecum) was removed. The removed digestive tract was cut along the midline and transferred to a 50 mL polypropylene tube on ice, and then 10 mL of saline was added. The container was sealed and centrifuged at 4°C at 3000 rpm for 10 minutes, and the supernatant was regarded as an intestinal lavage fluid.

Next, 153 mg of AST-120 (fine granules), which is a type of spherical carbon adsorbent, was weighed, suspended in 120 mL of the intestinal lavage fluid or saline, and subjected to reciprocal stirring at 37°C overnight at 74 rpm. Then, about 5, 10, 20, 30, 40, 60, 80, and 100 mg were weighed in terms of wet weight, and adsorption performance evaluations were performed. Adsorption performance evaluations were performed by dissolving indole, which is a precursor of uremic-toxin indoxyl sulfate, in a 50 mM phosphate buffer (pH 7.2) so as to have a concentration of 0.1 g/L, adding 40 mL of the solution to AST-120 weighed as described above, subjecting the mixture to reciprocal stirring in a sealed container at 37°C for 3 hours at 74 rpm, and then measuring the indole concentration in the supernatant.

As a result of measurement, it was found that the amount of indole adsorbed of the intestinal lumen fluid group was smaller than that of the control saline group, and the adsorption performance of AST-120 was reduced by exposure to the extract (intestinal lavage fluid) from the digestive tract above the large intestine.

### (Reference Example 2) Adsorption performance evaluation of spherical carbon adsorbent in digestive tract

0.5 mL of AST-120 (fine granules, 1 g/5 mL suspension for oral administration prepared from 1.25% tragacanth) was orally administered to male Crlj:ZUC-Lepr[fa] rats at 10 weeks old, and the rats were euthanized with carbon dioxide after 50 minutes. Subsequently, AST-120 was recovered from the digestive tract and regarded as an after-administration sample. The suspension for administration before oral administration was regarded as a before-administration sample.

Adsorption performance evaluations were performed by dissolving indole in a 50 mM phosphate buffer (pH 7.2) so as to have a concentration of 0.1 g/L, adding 40 mL of the solution to AST-120 weighed in advance in the range of 10 to 240 mg, subjecting the mixture to reciprocal stirring in a sealed container at 37°C for 3 hours at 74 rpm, and then measuring the indole concentration in the supernatant.

As a result of measurement, it was found that the amount of indole adsorbed of the after-administration sample was smaller than that of the before-administration sample, and the adsorption performance of AST-120 was reduced after oral administration at a stage before reaching the large intestine.

### (Example 1) Production of capsule preparation wherein capsule coated with enteric polymer is filled with spherical carbon adsorbent (Formulation Example 1)

Size-5 gelatin capsules (Capsugel Japan) were manually filled with 100 mg of AST-120 (fine granules).

The resulting capsules were coated based on the following organic solvent-based formulation. A subcoating of hydroxypropyl cellulose (HPC-SSL) was used on the gelatin capsules (the composition of the subcoating solution is shown in Table 1-1). The subcoated gelatin capsules were coated with a preparation solution (the composition is shown in Table 1-2) containing Eudragit S100, which is an enteric polymer. The Freund HC-Lab was used as a coater. The production conditions at the time of coating are as shown in Table 2, and the formulation of the capsule portion of the resulting pharmaceutical preparation is as shown in Table 3.

**(Table 1-1)**

| Subcoating solution component | Amount (g) |
|---|---|
| Hydroxypropyl cellulose (HPC-SSL) | 9.2 |
| Ethanol | 105.8 |
| Total | 115.0 |

**(Table 1-2)**

| Enteric polymer coating solution component | Amount (g) |
|---|---|
| Eudragit S100 | 69.3 |
| Triethyl citrate | 6.9 |
| Talc | 34.8 |
| Ethanol | 999.0 |
| Total | 1110.0 |

**(Table 2)**

| Production conditions: | |
|---|---|
| Set temperature of inlet air (°C) | 35 |
| Temperature of outlet air (°C) | 28 to 32 |
| Flow rate of inlet air (m³/min) | 1.2 to 1.3 |
| Amount of sprayed solution (g/min) | 2.3 to 6.6 |
| Air pressure (MPa) | 0.2 |

**(Table 3)**

| Capsule formulation component | Weight (mg) | Formulation proportion (%) |
|---|---|---|
| Gelatin capsule | 40.0 | 25.0 |
| Hydroxypropyl cellulose (HPC-SSL) | 9.2 | 5.7 |
| Eudragit S100 | 69.3 | 43.3 |
| Triethyl citrate | 6.9 | 4.3 |
| Talc | 34.8 | 21.7 |
| Total | 160.2 | 100.0 |

### (Example 2) Production of combination capsule preparation

### (Formulation Examples 2 to 3)

One size-0 capsule was filled with 2 pieces of the capsule preparation produced in Example 1 and 2 mg of enalapril or 10 mg of losartan to give a combination capsule preparation. (Formulation Example 2: enalapril-containing capsule preparation, Formulation Example 3: losartan-containing capsule preparation)

### (Example 3) Production of granule preparation wherein spherical carbon adsorbent is coated with enteric polymer

As for the respective granule preparations produced, the type of spherical carbon adsorbent used, the type of enteric polymer used for coating, and the amount of coating (% by weight in terms of polymer solids relative to spherical carbon adsorbent) are summarized in Table 4.

**(Table 4)**

| Number | Spherical carbon adsorbent | Enteric polymer (composition) | Amount of coating |
|---|---|---|---|
| Formulation Example 4 | AST-120 | Eudragit S100 (Table 5) | 15% |
| Formulation Example 5 | AST-120 | Eudragit S100 (Table 5) | 20% |
| Formulation Example 6 | AST-120 | Eudragit S100 (Table 5) | 30% |
| Formulation Example 7 | AST-120 | Eudragit S100 (Table 5) | 40% |
| Formulation Example 8 | AST-120 | Eudragit S100:L100=1:1 (Table 7) | 15% |
| Formulation Example 9 | AST-120 | Eudragit S100:L100=1:1 (Table 7) | 20% |
| Formulation Example 10 | AST-120 | Eudragit S100:L100=1:1 (Table 7) | 30% |
| Formulation Example 11 | AST-120 | Eudragit S100:L100=1:1 (Table 7) | 40% |
| Formulation Example 12 | AST-120 | Eudragit S100:L100=3:1 (Table 10) | 30% |
| Formulation Example 13 | Spherical carbon adsorbent "Mylan" | Eudragit S100:L100=3:1 (Table 10) | 30% |
| Formulation Example 14 | Spherical carbon adsorbent "Nichiiko" | Eudragit S100:L100=3:1 (Table 10) | 30% |
| Formulation Example 15 | AST-120 | Eudragit L30D55 (Table 13) | 10% |
| Formulation Example 16 | AST-120 | Eudragit L30D55 (Table 13) | 30% |
| Formulation Example 17 | AST-120 | Eudragit L30D55 (Table 13) | 60% |
| Formulation Example 18 | AST-120 | Eudragit L30D55 (Table 13) | 100% |
| Formulation Example 19 | AST-120 | Eudragit S100 (Table 15) | 30% |
| Formulation Example 20 | AST-120 | Eudragit S100:L100=1:1 (Table 17) | 30% |
| Formulation Example 21 | AST-120 | HPMC-AS-HG(Table 19) | 10% |
| Formulation Example 22 | AST-120 | HPMC-AS-HG(Table 19) | 17% |

In the following Formulation Examples, Powrex FD-MP-01 was used as a coater for preparing the samples.

### Formulation Examples 4 to 11:

AST-120 was coated based on the following solvent-based formulation. As an enteric polymer, Eudragit S100 (the composition of the coating solution is shown in Table 5) or a base obtained by mixing Eudragit L100 and Eudragit S100 in a ratio of 1:1 (the composition of the coating solution is shown in Table 7) was used. Samples were coated to 15%, 20%, 30%, and 40% in terms of polymer solids relative to AST-120. The formulations of pharmaceutical preparations coated to 40% (Formulation Examples 7 and 11) are shown in Table 6 and Table 8. The production conditions at the time of coating are shown in Table 9.

**(Table 5)**

| Enteric polymer coating solution (S) component | Amount (g) |
|---|---|
| Eudragit S100 | 280.0 |
| Triethyl citrate | 28.0 |
| Talc | 140.0 |
| Ethanol | 4032.0 |
| Total | 4480.0 |

**(Table 6) (Formulation of Formulation Example 7)**

| Granule preparation formulation (S) component | Weight (mg) | Formulation proportion (%) |
|---|---|---|
| AST-120 | 1000.0 | 61.0 |
| Eudragit S100 | 400.0 | 24.4 |
| Triethyl citrate | 40.0 | 2.4 |
| Talc | 200.0 | 12.2 |
| Total | 1640.0 | 100.0 |

**(Table 7)**

| Enteric polymer coating solution (S+L) component | Amount (g) |
|---|---|
| Eudragit L100 | 140.0 |
| Eudragit S100 | 140.0 |
| Triethyl citrate | 28.0 |
| Talc | 140.0 |
| Ethanol | 4032.0 |
| Total | 4480.0 |

**(Table 8) (Formulation of Formulation Example 11)**

| Granule preparation formulation (S+L) component | Weight (mg) | Formulation proportion (%) |
|---|---|---|
| AST-120 | 1000.0 | 61.0 |
| Eudragit L100 | 200.0 | 12.2 |
| Eudragit S100 | 200.0 | 12.2 |
| Triethyl citrate | 40.0 | 2.4 |
| Talc | 200.0 | 12.3 |
| Total | 1640.0 | 100.0 |

**(Table 9)**

| Production conditions: | |
|---|---|
| Set temperature of inlet air (°C) | 31 to 45 |
| Temperature of outlet air (°C) | 26 to 31 |
| Flow rate of inlet air (m³/min) | 0.6 to 0.9 |
| Amount of sprayed solution (g/min) | 2 to 6 |
| Air pressure (MPa) | 0.2 |

### Formulation Examples 12 to 14:

AST-120, spherical carbon adsorbent "Mylan", and spherical carbon adsorbent "Nichiiko" were coated based on the following solvent-based formulation. As an enteric polymer, a base obtained by mixing Eudragit L100 and Eudragit S100 in a ratio of 1:3 (the composition of the coating solution is shown in Table 10) was used. Samples were coated to 30% in terms of polymer solids relative to each spherical carbon adsorbent. The formulations of pharmaceutical preparations coated to 30% (Formulation Examples 12 to 14) are shown in Table 11. The production conditions at the time of coating are shown in Table 12.

**(Table 10)**

| Enteric polymer coating solution (S+L) component | Amount (g) |
|---|---|
| Eudragit L100 | 37.5 |
| Eudragit S100 | 112.5 |
| Triethyl citrate | 15.0 |
| Talc | 75.0 |
| Ethanol | 2160.0 |
| Total | 2400.0 |

**(Table 11) (Formulations of Formulation Examples 12 to 14)**

| Granule preparation formulation (S+L) component | Weight (mg) | Formulation proportion (%) |
|---|---|---|
| Spherical carbon adsorbent | 500.0 | 67.6 |
| Eudragit L100 | 37.5 | 5.1 |
| Eudragit S100 | 112.5 | 15.2 |
| Triethyl citrate | 15.0 | 2.0 |
| Talc | 75.0 | 10.1 |
| Total | 740.0 | 100.0 |

**(Table 12)**

| Production conditions: | |
|---|---|
| Set temperature of inlet air (°C) | 37 to 45 |
| Temperature of outlet air (°C) | 28 to 29 |
| Flow rate of inlet air (m³/min) | 0.7 to 1.0 |
| Amount of sprayed solution (g/min) | 2 to 6 |
| Air pressure (MPa) | 0.2 |

### Formulation Examples 15 to 22:

AST-120 was coated based on the following solvent-based formulations. As enteric polymers, Eudragit L30D55 (the composition of the coating solution is shown in Table 13), Eudragit S100 (the composition of the coating solution is shown in Table 15), Eudragit S100:L100 = 1:1 (the composition of the coating solution is shown in Table 17), and HPMC-AS-HG (the composition of the coating solution is shown in Table 19) were used. Samples were coated to 10 to 100% in terms of polymer solids relative to AST-120 (as shown in Table 4). The formulations of pharmaceutical preparations obtained by coating AST-120 with Eudragit L30D55, Eudragit S100, and Eudragit S100:L100 = 1:1 to 30% (Formulation Example 16, Formulation Example 19, and Formulation Example 20) are shown in Table 14, Table 16, and Table 18. The formulation of a pharmaceutical preparation obtained by coating AST-120 with HPMC-AS-HG to 17% (Formulation Example 22) is shown in Table 20. The production conditions at the time of coating are shown in Table 21.

**(Table 13)**

| Enteric polymer coating solution component (L30D55) | Amount (g) |
|---|---|
| Eudragit L130D55 | 1036.3 |
| (in terms of solid content) | 310.9 |
| Triethyl citrate | 31.1 |
| Talc | 155.4 |
| Water | 777.2 |
| Total | 2000.0 |

**(Table 14) (Formulation of Formulation Example 16)**

| Granule preparation formulation component (L30D55) | Weight (mg) | Formulation proportion (%) |
|---|---|---|
| Spherical carbon adsorbent | 400.0 | 51.8 |
| Eudragit L30D55 (solid content) | 120.0 | 15.5 |
| Triethyl citrate | 3.6 | 1.6 |
| Talc | 18.0 | 7.8 |
| Total | 541.6 | 100.0 |

**(Table 15)**

| Enteric polymer coating solution (S) component | Amount (g) |
|---|---|
| Eudragit S100 | 200.0 |
| Triethyl citrate | 100.0 |
| Talc | 100.0 |
| 1 N Aqueous ammonia solution | 136.0 |
| Water | 1464.0 |
| Total | 2000.0 |

**(Table 16) (Formulation of Formulation Example 19)**

| Granule preparation formulation (S) component | Weight (mg) | Formulation proportion (%) |
|---|---|---|
| AST-120 | 400.0 | 62.5 |
| Eudragit S100 | 120.0 | 18.8 |
| Triethyl citrate | 60.0 | 9.4 |
| Talc | 60.0 | 9.4 |
| Total | 640.0 | 100.0 |

**(Table 17)**

| Enteric polymer coating solution (S+L) component | Amount (g) |
|---|---|
| Eudragit L100 | 100.0 |
| Eudragit S100 | 100.0 |
| Triethyl citrate | 100.0 |
| Talc | 100.0 |
| 1 N Aqueous ammonia solution | 128.0 |
| Water | 1472.0 |
| Total | 2000.0 |

**(Table 18) (Formulation of Formulation Example 20)**

| Granule preparation formulation (S+L) component | Weight (mg) | Formulation proportion (%) |
|---|---|---|
| AST-120 | 400.0 | 62.5 |
| Eudragit L100 | 60.0 | 9.4 |
| Eudragit S100 | 60.0 | 9.4 |
| Triethyl citrate | 60.0 | 9.4 |
| Talc | 60.0 | 9.4 |
| Total | 640.0 | 100.0 |

**(Table 19)**

| Enteric polymer coating solution component HPMC-AS-HG | Amount (g) |
|---|---|
| HPMC-AS-HG | 180.0 |
| Ethanol | 2256.0 |
| Water | 564.0 |
| Total | 3000.0 |

**(Table 20) (Formulation of Formulation Example 22)**

| Granule preparation formulation component HPMC-AS-HG | Weight (mg) | Formulation proportion (%) |
|---|---|---|
| Spherical carbon adsorbent | 300.0 | 76.9 |
| HPMC-AS-HG | 90.0 | 23.1 |
| Total | 390.0 | 100.0 |

**(Table 21)**

| Production conditions: | |
|---|---|
| Set temperature of inlet air (°C) | 35 to 50 |
| Temperature of outlet air (°C) | 26 to 35 |
| Flow rate of inlet air (m³/min) | 0.3 to 1.1 |
| Amount of sprayed solution (g/min) | 2 to 5 |
| Air pressure (MPa) | 0.5 |

### (Example 4) Adsorption performance evaluation of spherical carbon adsorbent coated with enteric polymer and uncoated spherical carbon adsorbent

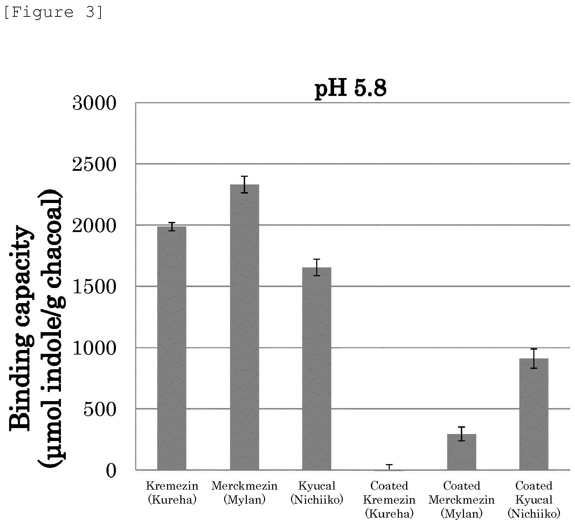

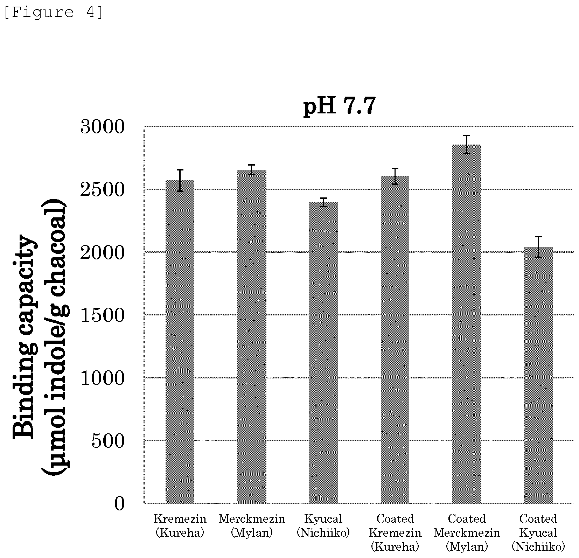

The adsorption performances of spherical carbon adsorbent coated with an enteric polymer (granule preparations produced in Example 3: Formulation Examples 4 to 11) and uncoated spherical carbon adsorbent (AST-120 fine granules) were evaluated in two pH conditions, i.e., an acidic to weakly acidic region (pH 5.6) mimicking the upper part of the digestive tract and a neutral region (pH 7.8) mimicking the lower part of the digestive tract. As for the amount of indole adsorbed, indole was dissolved in a 400 mM MES buffer (pH 5.6) or a 50 mM phosphate buffer (pH 7.8) so as to have a concentration of 0.1 g/L, 40 mL of the solution was added to each of the spherical carbon adsorbent coated with an enteric polymer weighed in advance in the range of 9 to 15 mg and uncoated spherical carbon adsorbent, each mixture was subjected to reciprocal stirring in a sealed container at 37°C for 3 hours at 74 rpm, and then the indole concentration in the supernatant was measured. After measuring the indole concentration in the supernatant, the weight of precipitates (the spherical carbon adsorbent coated with an enteric polymer and uncoated spherical carbon adsorbent) was measured, and the adsorption performance was determined by dividing the amount of adsorbed indole by the weight of precipitates. Results are shown in Figure 1 (pH 5.6) and Figure 2 (pH 7.8). Also, the adsorption performance was determined in the same manner as above except that spherical carbon adsorbent coated with an enteric polymer (the granule preparations produced in Example 3: Formulation Examples 12 to 14) was used, and a 400 mM MES buffer (pH 5.8) and a 50 mM phosphate buffer (pH 7.7) were used as buffers. Results are shown in Figure 3 (pH 5.8) and Figure 4 (pH 7.7) .

It was shown that the granule preparations of the present invention (spherical carbon adsorbent coated with an enteric polymer) are masked to a level such that the adsorption performance is low in acidic to weakly acidic conditions that mimic the upper part of the digestive tract and, on the other hand, the intrinsic adsorption performance is recovered in a neutral environment that mimics the lower part of the digestive tract where uremic toxins derived from intestinal bacteria and precursors thereof are present in large amounts.

### (Example 5) In vivo evaluation test (1): Influence on blood level of simultaneously administered drug (enalapril)

The enalapril-containing spherical carbon adsorbent capsule preparation produced in Example 2 (Formulation Example 2) was orally administered to Cynomolgus monkeys one capsule per animal (5 animals in total). Blood was collected before oral administration and 0.5, 1, 2, 4, 8, and 24 hours after administration, the plasma enalaprilat (active metabolite of enalapril) level was measured, and the average of 5 animals was summarized in a graph. As a comparative control, a combination capsule preparation was used that was produced in the same manner as in Example 3 except that size-5 capsules not coated with an enteric polymer were filled with spherical carbon adsorbent. Also, in order to measure the plasma enalaprilat level when enalapril was administered singly, one size-0 capsule filled solely with enalapril was administered. Results are shown in Figure 5.

It was shown that spherical carbon adsorbent when administered simultaneously with enalapril, which is often prescribed for kidney disease patients, reduces the blood level thereof, whereas the pharmaceutical preparation of the present invention even when administered simultaneously with enalapril enables the blood level to be maintained at the same level as a level attained when the drug is administered singly.

### (Example 6) In vivo evaluation test (2): Influence on blood level of simultaneously administered drug (losartan)

Example 6 was performed in the same manner as Example 5 by using the losartan-containing spherical carbon adsorbent capsule preparation produced in Example 2 (Formulation Example 2). Note that the dosage of losartan was 10 mg, and the plasma level of losartan was measured. Results are shown in Figure 6.

It was shown that spherical carbon adsorbent when administered simultaneously with losartan, which is often prescribed for kidney disease patients, reduces the blood level, whereas the capsule preparation of the present invention even when administered simultaneously with losartan enables the blood level to be maintained at the same level as a level attained when the drug is administered singly.

### (Example 7) In vivo evaluation test (3): Influence on plasma uremic toxin level

In this test, among the granule preparations produced in Example 3, the granule preparation (Formulation Example 7) coated with Eudragit S100 as an enteric polymer to a ratio of 40% in terms of polymer solids relative to AST-120 was used. Dosing solutions were prepared by suspending the granule preparation and uncoated spherical carbon adsorbent (AST-120 (fine granules)) each in an amount of 0.3 g in terms of the weight of spherical carbon adsorbent in 10 mL of a 1.25% tragacanth solution. 10 mL each of the dosing solutions was orally administered per 1 kg of male Crlj:ZUC-Lepr[fa] rats at 11 weeks old (N=5). The rats were fasted after administration, blood was collected from the caudal vein before oral administration and 1, 2, 4, and 6 hours after administration, the plasma level of indoxyl sulfate (IS), which is one of the uremic toxins, was measured, and the extent of reduction of plasma IS (Δ Plasma IS, mg/dL) from the time before oral administration was calculated. The average ± standard error of each group at each time point is shown in Figure 7. The granule preparation of the present invention (Formulation Example 7) showed a greater reduction of plasma indoxyl sulfate level than uncoated spherical carbon adsorbent.

### (Example 8) In vivo evaluation test (4): Dosage of spherical carbon adsorbent and influence on plasma uremic toxin level

In this test, among the granule preparations produced in Example 3, the granule preparation (Formulation Example 12) coated with Eudragits S100 and L100 (3:1) as enteric polymers to a ratio of 30% in terms of polymer solids relative to AST-120 was used. Dosing solutions were prepared by suspending the granule preparation and uncoated spherical carbon adsorbent (AST-120 (fine granules)) each in an amount of 100 mg or 300 mg in terms of the weight of spherical carbon adsorbent in 10 mL of a 1.25% tragacanth solution. 10 mL each of the dosing solutions was orally administered per 1 kg of SD(Slc:SD) rats (N=8 to 9). The rats were fasted after administration, and 2 hours after oral administration, 1 mL of a probenecid solution (50 mg/mL) was intravenously administered per 1 kg of the rats. Blood was collected from the vein before oral administration, before intravenous administration, and 1, 2, 3, and 4 hours after intravenous administration, the plasma level of indoxyl sulfate (IS), which is one of the uremic toxins, was measured, and the extent of reduction of plasma IS (Δ plasma IS, mg/dL) from the time before oral administration was calculated. The average ± standard error of each group at each time point is shown in Figure 8. The granule preparation of the present invention (Formulation Example 12) showed an identical reduction of plasma indoxyl sulfate level in a dosage 1/3 of uncoated spherical carbon adsorbent.

### (Example 9) In vivo evaluation test (5): Dosage of spherical carbon adsorbent and influence on plasma uremic toxin level (non-rodent animal)

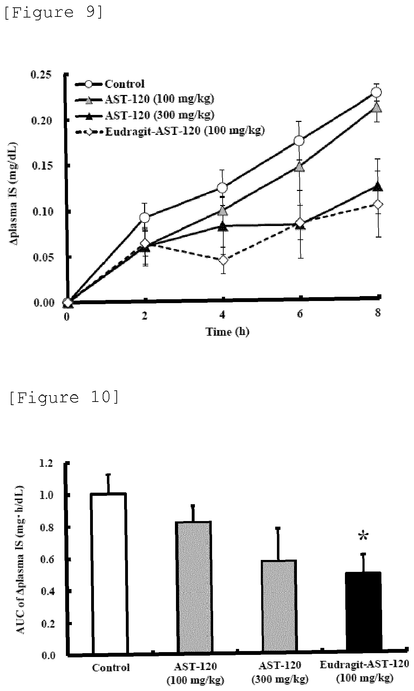

In this test, among the granule preparations produced in Example 3, the granule preparation (Formulation Example 12) coated with Eudragits S100 and L100 (3:1) as enteric polymers to a ratio of 30% in terms of polymer solids relative to AST-120 was used. Gelatin capsules (No. 12 (1/4 oz)) were filled respectively with the granule preparation and uncoated spherical carbon adsorbent (AST-120 (fine granules)) so as to be 100 mg or 300 mg in terms of the weight of spherical carbon adsorbent per 1 kg of Beagle dogs, and orally administered. 1 hour after oral administration, 2.5 mL of a probenecid solution (20 mg/mL) was orally administered per 1 kg of Beagle dogs. 2 hours after oral administration of spherical carbon adsorbent, the dogs were fed (DS-A, 250 g/dog) again. Blood was collected from the vein before feeding and 2, 4, 6, and 8 hours after feeding, the plasma level of indoxyl sulfate (IS), which is one of the uremic toxins, was measured, and the extent of reduction of plasma IS (Δ plasma IS, mg/dL and Δ plasma IS AUC (0 to 8 h), mg·h/dL) from the time before feeding was calculated. The average ± standard error of each group at each time point is shown in Figure 9, and Δ plasma IS AUC from before feeding (0 h) to 8 h is shown in Figure 10. The granule preparation of the present invention (Formulation Example 12) showed an identical reduction of plasma indoxyl sulfate level in a dosage 1/3 of uncoated spherical carbon adsorbent.

### (Example 10) In vivo evaluation test (6): Influence on blood uremic toxin level (when the type of spherical carbon adsorbent and the type/amount of coating of enteric polymer are changed)

In this test, among the granule preparations produced in Example 3, the granule preparations (Formulation Examples 15, 16, 12, 19, 22, and 13) coated with an enteric polymer shown in Table 22 to a ratio of 10 to 30% in terms of polymer solids relative to AST-120 (or relative to spherical carbon adsorbent fine granules "Mylan") were used. Dosing solutions were prepared by suspending the granule preparations and uncoated spherical carbon adsorbent (AST-120 (fine granules)) each in an amount of 100 mg in terms of the weight of spherical carbon adsorbent in 10 mL of a 1.25% tragacanth solution. 10 mL each of the dosing solutions was orally administered per 1 kg of SD(Slc:SD) rats (N=6 to 19). The rats were fasted after administration, and 2 hours after oral administration, 1 mL of a probenecid solution (50 mg/mL) was intravenously administered per 1 kg of the rats. Blood was collected from the vein before oral administration, before intravenous administration, and 1, 2, 3, and 4 hours after intravenous administration, the plasma level of indoxyl sulfate (IS), which is one of the uremic toxins, was measured, and the extent of reduction of plasma IS (Δ plasma IS, AUC (-2 h to 4 h)) from the time before oral administration was calculated. The ratio to the solvent control group (a 1.25% tragacanth solution administered group) being 1 was calculated, and the average of each group is shown in Table 22. The granule preparations of the present invention (Formulation Examples 15, 16, 12, 19, 22, and 13) showed a greater reduction of plasma indoxyl sulfate level than uncoated spherical carbon adsorbent.

**(Table 22)**

| Pharmaceutical preparation (Type of carbon adsorbent/coating, dissolution pH, amount) | N | ratio vs control |
|---|---|---|
| Control | 19 | 1.00 |
| AST-120 | 19 | 0.47 |
| Formulation Example 15 (AST-120, Eudragit_L30D55, pH5.5, 10%) | 6 | -0.36 |
| Formulation Example 16 (AST-120, Eudragit_L30D55, pH5.5, 30%) | 6 | -0.02 |
| Formulation Example 12 (AST-120, Eudragit_S100:L100=3:1, pH6.75, 30%) | 6 | 0.04 |
| Formulation Example 19 (AST-120, Eudragit_S100, pH7.0, 30%) | 7 | 0.33 |
| Formulation Example 22 (AST-120, HPMC-AS-HG, pH6.5, 17%) | 6 | 0.35 |
| Formulation Example 13 ("Mylan",Eudragit_S100:L100=3:1, pH6.75, 30%) | 6 | 0.20 |

### (Example 11) In vivo evaluation test (7): Verification of exposed state of spherical carbon adsorbent surface of pharmaceutical preparation of present invention in small intestine and large intestine

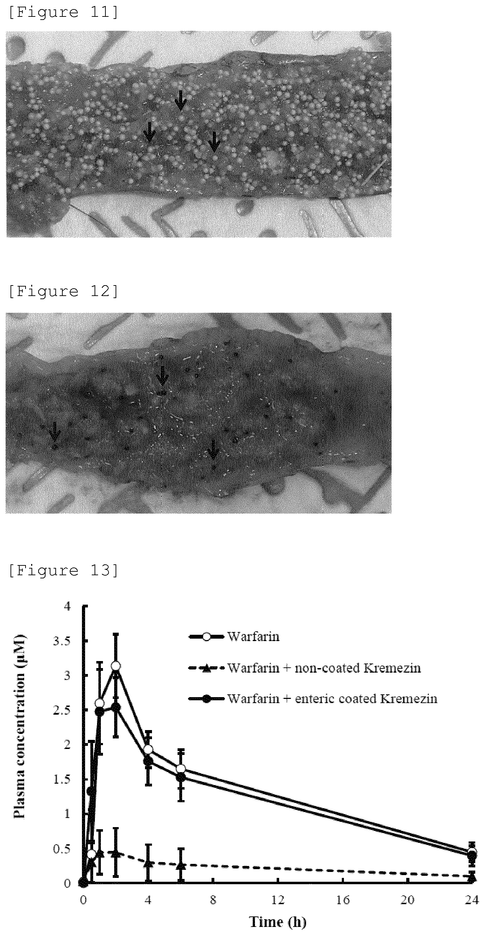

In this test, among the granule preparations produced in Example 3, the granule preparation (Formulation Example 7) coated with Eudragit S100 as an enteric polymer to a ratio of 40% in terms of polymer solids relative to AST-120 was used. 0.3 g of the granule preparation in terms of weight was weighed and suspended in 10 mL of a 1.25% tragacanth solution to prepare a dosing solution. After 10 mL of the dosing solution was orally administered per 1 kg of male Crlj:ZUC-Lepr[fa] rats at 11 weeks old, the rats were fasted, then euthanized with carbon dioxide after 1 and 4 hours, and subjected to laparotomy to observe the state of spherical carbon adsorbent in the digestive tract. At 1 hour after administration, spherical carbon adsorbent was present in the small intestine. This appeared grayish white as shown in Figure 11, and it was verified by observation with the naked eye that the coating was maintained. At 4 hours after administration, spherical carbon adsorbent was present in the large intestine, the coating dissolved, and spherical carbon adsorbent returned to black. It was thus verified that AST-120, which is the content, was exposed (Figure 12). Accordingly, it was shown that the enteric polymer does not dissolve in the small intestine but dissolves in the large intestine, and AST-120, which is the content, is exposed in the large intestine.

### (Example 12) In vivo evaluation test (8): Influence on blood level of simultaneously administered drug (warfarin) of granule preparation wherein spherical carbon adsorbent is coated with enteric polymer

A granule preparation produced in the same manner as in Formulation Example 12 of Example 3 (Eudragits S100 and L100 (3:1) as enteric polymers) was used. One size-0 capsule filled with 171±4 mg of the granule preparation coated to a ratio of 30% relative to AST-120 (produced using the formulation of Table 11 and the production conditions described in Table 9) and 0.5 mg of warfarin (0.5 mg of warfarin content obtained by grinding a 5 mg warfarin tablet) was orally administered per Cynomolgus monkey (6 animals in total). Blood was collected before oral administration and 0.5, 1, 2, 4, 8, and 24 hours after administration, the plasma warfarin level was measured, and the average of 6 animals was summarized in a graph. Eight days after the administration described above, one size-0 capsule filled with granular AST-120 (120±2 mg) not coated with an enteric polymer and 0.5 mg of warfarin (0.5 mg of warfarin content obtained by grinding a 5 mg warfarin tablet) was orally administered as a comparative control and, similarly, blood was collected to measure the plasma warfarin level, and the average of 6 animals was summarized in a graph. Furthermore, in order to measure the plasma warfarin level when warfarin is administered singly, 12 days after the administration described above, one size-0 capsule filled with 0.5 mg of warfarin (0.5 mg of warfarin content obtained by grinding a 5 mg warfarin tablet) was orally administered and, similarly, blood was collected to measure the plasma warfarin level, and the average of 6 animals was summarized in a graph. The results of total 3 times are shown in Figure 13.

It was shown that spherical carbon adsorbent when administered simultaneously with warfarin, which is often prescribed for kidney disease patients, reduces the blood level thereof, whereas the pharmaceutical preparation of the present invention even when administered simultaneously with warfarin enables the blood level to be maintained at the same level as a level attained when the drug is administered singly.

### (Example 13) Adsorption test: Adsorption performance evaluation using methylene blue of granules wherein spherical carbon adsorbent is coated with enteric polymer

The granule preparations of Formulation Example 12 and Formulation Examples 13 and 15 to 22 corresponding to 50 mg in terms of spherical carbon adsorbent were weighed and used in the test.

Test solutions having a pH of 1.2 (Japanese Pharmacopoeia dissolution test 1st solution JP1) and a pH of 7.5 (McIlvaine buffer) were used. The test solutions were adjusted such that the concentration of methylene blue, which is an adsorbate, was 40 mg/L. Methylene blue absorbance was measured using a dissolution apparatus by a paddle method at 200 rpm in 500 mL of a test solution at a UV measurement wavelength of 246 nm (the test time is 2 hours at a pH of 1.2 and 6 hours at a pH of 7.5). In reference to the JIS activated carbon test, the amount of methylene blue adsorbed on 1 g of spherical carbon adsorbent was calculated.

The results of the test performed using Formulation Example 12 and Formulation Examples 13 and 15 to 22 are shown in Table 23. In the adsorption test using methylene blue, it was verified that the pharmaceutical preparations adsorbed methylene blue in an amount of less than 50 mg/g in 2 hours in a test solution having a pH of 1.2, and adsorbed methylene blue in an amount of 80 mg/g or more in 6 hours in a test solution having a pH of 7.5. These results show that the pharmaceutical preparation coated with an enteric polymer of the present invention is coated until reaching the target site (the large intestine or a lower part of the small intestine) and thus functions to maintain the intrinsic adsorption performance without being reduced and demonstrate adsorption performance for the first time after reaching the target site.

**(Table 23)**

| Number | Amount of coating | Methylene blue adsorption test (mg/g) | |
|---|---|---|---|
| | | pH1.2 | pH7.5 |
| Formulation Example 12 | 30% | 10.9 | 173.2 |
| Formulation Example 13 | 30% | 20.5 | 357.0 |
| Formulation Example 15 | 10% | -0.6 | 143.3 |
| Formulation Example 16 | 30% | -4.1 | 191.0 |
| Formulation Example 17 | 60% | 23.6 | 156.1 |
| Formulation Example 18 | 100% | 3.6 | 142.1 |
| Formulation Example 19 | 30% | 14.1 | 209.0 |
| Formulation Example 20 | 30% | 0.2 | 124.0 |
| Formulation Example 21 | 10% | 10.1 | 115.3 |
| Formulation Example 22 | 17% | 11.4 | 168.3 |

### Industrial Applicability

The pharmaceutical preparation of the present invention does not affect the blood level of a concomitant drug even when simultaneously administered with the concomitant drug and has a higher toxic substance adsorbability in the living body than a conventional spherical carbon adsorbent, therefore the volume when administered can be reduced, and the amount of water consumption can also be reduced. Accordingly, the pharmaceutical preparation is effective for reducing blood uremic toxins, improving uremic symptoms, delaying dialysis initiation, or protecting renal functions in a patient with CKD.

## Claims

1. A pharmaceutical preparation for oral administration comprising a porous material, wherein a means for exposing a surface of the porous material for the first time in the large intestine or a lower part of the small intestine is provided on the porous material.

2. A pharmaceutical preparation according to claim 1, wherein an amount of an adsorbate adsorbed in an adsorption test of the pharmaceutical preparation is less than 20% in an environment having a pH of 5 or less and 60% or more in an environment having a pH of 7 or more based on an amount of an adsorbate adsorbed by a porous material not provided with the means.

3. A pharmaceutical preparation according to claim 1 or 2, wherein in an adsorption test using methylene blue as an adsorbate, when the pharmaceutical preparation (an amount corresponding to 50 mg of the porous material when the pharmaceutical preparation is a granule preparation, one tablet when the pharmaceutical preparation is a tablet preparation, and an amount corresponding to one capsule when the pharmaceutical preparation is a capsule preparation) is tested in a dissolution apparatus using 500 mL of a test solution having a methylene blue concentration of 40 mg/L, an amount of the adsorbate adsorbed in 2 hours in the test solution having a pH of 1.2 is less than 50 mg per 1 g of the porous material, and an amount of the adsorbate adsorbed in 6 hours in the test solution having a pH of 7.5 is 80 mg or more per 1 g of the porous material.

4. A pharmaceutical preparation according to any one of claims 1 to 3, wherein the pharmaceutical preparation wherein the means for exposing a surface of the porous material for the first time in the large intestine or a lower part of the small intestine is provided is selected from a) to c) below:
a) a granule preparation, a capsule preparation, or a tablet preparation comprising a porous material coated with an enteric polymer;
b) a capsule preparation wherein a porous material is encapsulated within an enteric capsule; and
c) a tablet preparation wherein a compression-molded product of a porous material is coated with an enteric polymer.

5. A pharmaceutical preparation according to any one of claims 1 to 4, wherein the porous material is an oral adsorbent for a harmful substance.

6. A pharmaceutical preparation according to any one of claims 1 to 5, wherein the porous material is spherical carbon adsorbent.

7. A pharmaceutical preparation according to any one of claims 1 to 6, wherein before the means is provided, the porous material has an average particle size X50 of 100 to 500 µm as measured by laser diffractometry and a specific surface area of 800 m²/g or more as measured by a BET multipoint method.

8. A pharmaceutical preparation according to any one of claims 1 to 7, wherein before the means is provided, the porous material has an average particle size X50 of 200 to 400 µm as measured by laser diffractometry and a specific surface area of 1000 to 1700 m²/g as measured by a BET multipoint method.

9. A pharmaceutical preparation according to any one of claims 1 to 8, wherein the enteric polymer or the enteric capsule dissolves at a pH of 5 to 8.

10. A pharmaceutical preparation according to any one of claims 1 to 9, wherein the enteric polymer is an acrylic-based polymer or a cellulose-based polymer.

11. A pharmaceutical preparation according to any one of claims 1 to 10, wherein the enteric polymer is one or more selected from the group consisting of a copolymer of methacrylic acid and ethyl acrylate, a copolymer of methacrylic acid and methyl methacrylate, a copolymer of methacrylic acid, methyl acrylate, and methyl methacrylate, cellulose acetate phthalate, cellulose acetate trimellitate, hydroxypropyl methylcellulose phthalate, hydroxypropyl methylcellulose acetate succinate, carboxymethyl ethylcellulose, cellulose acetate succinate, methylcellulose phthalate, hydroxymethylcellulose phthalate, hydroxypropyl methyl acetate maleate, hydroxypropyl methyl trimellitate, polyvinyl acetate phthalate, and polyvinyl butyrate phthalate.

12. A pharmaceutical preparation according to any one of claims 1 to 11, wherein the enteric polymer is one or more selected from methacrylic acid copolymer L, methacrylic acid copolymer S, methacrylic acid copolymer LD, and hydroxypropyl methylcellulose acetate succinate.

13. A pharmaceutical preparation according to any one of claims 1 to 12, wherein the pharmaceutical preparation wherein the means for exposing a surface of the porous material for the first time in the large intestine or a lower part of the small intestine is provided is a) a granule preparation, a capsule preparation, or a tablet preparation comprising a porous material coated with an enteric polymer.

14. A pharmaceutical preparation according to claim 13, wherein a coating of the enteric polymer is 10 to 100% by weight based on the porous material.

15. A pharmaceutical preparation according to claim 13, wherein a coating of the enteric polymer is 10 to 60% by weight based on the porous material.

16. A pharmaceutical preparation according to any one of claims 1 to 15, wherein
the pharmaceutical preparation wherein the means for exposing a surface of the porous material for the first time in the large intestine or a lower part of the small intestine is provided is a granule preparation or a capsule preparation comprising a porous material coated with an enteric polymer;
the porous material is spherical carbon adsorbent;
the enteric polymer is one or more selected from the group consisting of methacrylic acid copolymer L, methacrylic acid copolymer S, methacrylic acid copolymer LD, and hydroxypropyl methylcellulose acetate succinate; and an amount of a coating of the enteric polymer is 10 to 60% by weight based on the porous material.

17. A pharmaceutical preparation according to any one of claims 1 to 15, wherein
the pharmaceutical preparation wherein the means for exposing a surface of the porous material for the first time in the large intestine or a lower part of the small intestine is provided is a granule preparation or a capsule preparation comprising a porous material coated with an enteric polymer;
the porous material is spherical carbon adsorbent;
the enteric polymer is one or more selected from the group consisting of methacrylic acid copolymer L and methacrylic acid copolymer S; and
an amount of a coating of the enteric polymer is 10 to 60% by weight based on the porous material.

18. A pharmaceutical preparation according to any one of claims 1 to 17, for reducing a blood uremic toxin, improving a uremic symptom, delaying dialysis initiation, or protecting a renal function in a patient with chronic kidney disease.

19. A pharmaceutical preparation according to any one of claims 1 to 18, for simultaneous administration with a further drug.

20. A pharmaceutical preparation according to any one of claims 1 to 19, further comprising a further drug.

21. A pharmaceutical preparation according to claim 19 or 20, wherein the further drug is one or more selected from angiotensin II receptor blockers, angiotensin converting enzyme inhibitors, calcium antagonists, diuretics, hyperuricemia drugs, hyperlipidemia drugs, diabetes drugs, steroids/immunosuppressants, antiplatelet drugs/anticoagulants, hyperphosphatemia drugs, erythropoietic stimulating agents, analgesics, antiarrhythmic drugs, antidepressants, Alzheimer-type dementia drugs, Parkinson's disease drugs, proton pump inhibitors (PPIs), antiallergic drugs, and antibacterial drugs.

22. A pharmaceutical preparation according to claim 19 or 20, wherein the further drug is one or more selected from angiotensin II receptor blockers, angiotensin converting enzyme inhibitors, calcium antagonists, diuretics, and antiplatelet drugs/anticoagulants.

23. A method for producing the pharmaceutical preparation for oral administration of claims 1 to 22, the method comprising any of the following steps:
a) a step of coating a porous material with an enteric polymer;
b) a step of encapsulating a porous material within an enteric capsule; and
c) a step of coating a compression-molded product of a porous material with an enteric polymer.
